(19) Europäisches Patentamt
European Patent Office
Office européen des brevets

(11) Publication number: **0 414 384 A2**

# (12) EUROPEAN PATENT APPLICATION

(21) Application number: **90308196.6**

(22) Date of filing: **26.07.90**

(51) Int. Cl.5: **C07C 69/30**, C07C 67/08, C07C 69/52, C07C 69/587, C07C 69/533, A23L 1/29, A23D 9/00

(30) Priority: **08.08.89 US 390725**

(43) Date of publication of application: **27.02.91 Bulletin 91/09**

(84) Designated Contracting States: **AT BE CH DE DK ES FR GB GR IT LI LU NL SE**

(71) Applicant: **NABISCO BRANDS, INC.**
**200 Deforest Avenue**
**East Hanover NJ 07936-1944(US)**

(72) Inventor: **Klemann, Lawrence P.**
**196 Tanglewood Drive**
**Somerville, New Jersey 08876(US)**
Inventor: **Finley, John W.**
**3 Old Stone Lane**
**Whippany, New Jersey 07981(US)**
Inventor: **Scimone, Anthony**
**14 Sweetwood Drive**
**Cedar Grove, New Jersey 07009(US)**

(74) Representative: **Thomas, Roger Tamlyn et al**
**D. Young & Co. 10 Staple Inn**
**London WC1V 7RD(GB)**

## (54) Process for preparing neoalkyl triol triesters.

(57) Edible fat mimetics, neoalkyl triol triesters, are prepared in high yield in a simple, economical thermal fusion between one molar equivalent of *tris*hydroxymethyl lower alkane triol and a slight excess of three molar equivalents of fatty acid.

EP 0 414 384 A2

# PROCESS FOR PREPARING NEOALKYL TRIOL TRIESTERS

## BACKGROUND OF THE INVENTION

This invention relates to the preparation of neoalkyl triol triester edible fat mimetics, *tris*hydroxymethyl lower alkane fatty acid esters.

*Tris*hydroxymethyl ethane and propane fatty acid esters have been synthesized for use as high temperature lubricants (for engines and metal working; see reviews in Barnes, R.S., and Fainman, M.Z., 13 *Lub. Eng.* 454 (1957); Smith, T.G., chapter 10 of Gunderson, R.C., and Hart, A.W., eds., *Synthetic Lubricants*, Reinhold Pub. Corp., 1962, pages 388-401; and Bell, E.W., *et al.*, 53 *J. Amer. Oil Chem. Soc.* 511 (1976)). The two most generally accepted methods of manufacturing *tris*hydroxymethyl alkane ester synthetic lubricants are direct esterification of the neopentyl polyol with fatty acids, or transesterification between mono fatty acid esters and the polyol (Smith, *supra*, pages 390-391).

Smith, *ibid.*, summarizes reaction conditions for direct esterifications. The condensations are carried out in a solvent, commonly one that serves as an azeotropic agent to help carry off the water formed. A hydrocarbon boiling between about 80° and 150° C is generally employed for this purpose; toluene is often suggested (see, for example, U.S. Pat. No. 2,031,603 to Holt, col. 1, line 24 and Example 4; U.S. Pat. Pat. No. 3,282,971 to Metro, *et al.*, col. 2, line 54; and U.S. Pat. No. 3,464,922 to Bernholz and Cox, col. 2, line 42), or toluene or xylene (see, for example, U.S. Pat. No. 2,891,919 to Christenson and Borman, col. 2, line 62 (though the examples employ pentaerythritol rather than a *tris*hydroxy methyl lower alkane); and U.S. Pat. No. 3,441,600 to Chao, *et al.*, col. 3, line 10), but other azeotroping agents have been suggested, including heptane, cyclohexane, benzene, ethyl benzene, mesitylene, and mixtures of aliphatic hydrocarbons, such as petroleum naptha (U.S. Pat. No. 2,991,297 at col. 2, lines 16 to 19; U.S. Pat. No. 3,282,971 at col. 2, line 54; U.S. Pat. No. 3,562,300 to Chao and Kjonaas, col. 3, lines 60 to 63 (though the examples employ pentaerythritol exclusively); and U.S. Pat. No. 3,694,382 to Kleiman *et al.*, col. 3, lines 12 to 14).

Conventional acid reaction catalysts are employed to accelerate the reaction. Examples of catalysts that have been suggested include mineral acids, preferably sulfuric acid, hydrochloric acid, phosphoric acid and the like; acid salts, such as sodium bisulfate, potassium bisulfate, sodium bisulfite, calcium acetate, barium acetate, and the like: sulfonic acids, such as benzene sulfonic acid, toluene sulfonic acid, polystyrene sulfonic acid, methyl sulfonic acid, ethyl sulfonic acid, and the like; esters of titanium or zirconium, such as tetraethyl titanate, tetrabutyl titanate, tetra-n-propyl zirconate, and the like, or other organo-titanium or organo-tin compound having at least one organic (alkoxy, alkyl, dialkyl, aryloxy or alkylaryloxy) group attached to the metal atom, such as stannous octoate; and metal oxides, such as zinc oxide, alumina, and the like (U.S. Pat. No. 3,670,013 to Leibfried, col. 2, lines 69 to 75 (though the example employed pentaerythritol); U.S. Pat. No. 3,694,382 at col. 3, lines 22 to 32; U.S. Pat. No. 4,031,019 to Bell, col. 4, lines 6 to 7; and U.S. Pat. No. 4,234,497 to Honig, col. 3, lines 29 to 39).

The esterification reaction may, additionally, employ silicone antifoaming agents (U.S. Pat. No. 2,891,919 at col. 2, lines 70 to 71).

Solvents, catalysts, and other reagents employed in esterification reaçtions are difficult to separate from the final product, could contribute undesireable flavors and may even have toxic properties, limiting the usefulness of such syntheses in the food industry. Moreover, use of solvents, catalysts, and other reagents in addition to starting materials represents expense in storage, handling, and recovery, and necessitates complicated and costly purification steps subsequent to formation of reaction products.

Several improvements on catalyzed esterifications in a solvent have been suggested. U.S. Pat. Nos. 2,891,919, 2,991,297, and 3,441,600, cited *supra*, described esterifications carried out without catalysts, but all employ azeotroping agents. U.S. Pat. No. 3,620,290 to Kress, *et al.*, suggested removal of water by vacuum or gas sparging (col. 3, lines 59 to 63); the examples use both techniques (Examples 1 and 35), although xylene is used in an azeotropic distillation as well (Example 2) and catalysts are often employed (Examples 2 to 4). Removal of water by heating to high temperatures was attempted with dipentaerythritol (rather than neoalkyl triols, Example 5), but that procedure seemed to result in considerable decomposition, for the product is described as "viscous yellow brown" (col. 4, line 59). U.S. Pat. No. 3,670,013 to Leibfried, cited *supra*, suggested condensations in a thermal reaction zone, thereby avoiding solvents, but not catalysts, and the reaction products were partial, not full, esters. U.S. Pat. No. 4,317,780 to Mancini, *et al.* reported esterifications in a solvent-free media, but an acid catalyst was employed, and the reactions were carried out in an inert gas atmosphere (col. 3, lines 6 to 7). Brit. Pat. Nos. 951,938 and 951,939 to Heydon Newport (1964) used no catalysts (Example I of both patents), but xylene was employed, and reaction

conditions were complicated by the use of a carbon dioxide gas sparge and by the addition of activated carbon.

## SUMMARY OF THE INVENTION

In at least its preferred forms the present invention provides a process for preparing *tris*hydroxymethyl lower alkane fatty acid esters suitable for consumption, i.e., free of potentially toxic or noxious solvents, catalysts, and other reaction additives the process being efficient and economical.

This invention provides a process for the synthesis of neoalkyl triol triesters of the formula,

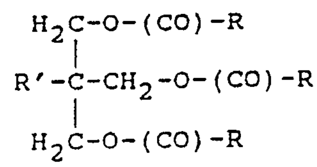

where

$R'$ is a lower alkyl group having 1 to 5 carbons, and each R is, independently, an aliphatic group having 1 to 29 carbons.

The trishydroxymethyl lower alkane ester products, which are generally edible fat mimetics, can be prepared in high yield by simple thermal fusion with the elimination of water. In the process of this invention, one molar equivalent of trishydroxymethyl lower alkane triol is preferably heated with a slight excess of three molar equivalents of fatty acid. Optionally, an acid catalyst can be employed.

Normally the reaction mixture will be heated until the reaction is substantially complete and the desired product is thereafter recovered.

Other aspects of the invention are set forth in the claims.

## DETAILED DESCRIPTION OF PREFERRED EMBODIMENTS

The following description relates to an improved synthesis of *tris*hydroxymethyl lower alkane esters suitable for incorporation into any food composition or use in conjunction with any edible material. The term "edible material" is broad and includes anything edible, whether or not intended for nutrition, e.g., it can be an additive such as an antioxidant for fats or oils, an antispatter agent, an emulsifier, a texture modifier such as a plasticizer for chewing gum, a component for cosmetics (e.g., lipstick), or other minor functional ingredient such as a carrier or diluent for use in flavorings, pharmaceuticals, and the like. Thus, chewing gum, flavored coatings, oils and fats intended only for frying, and the like are included. Representative of edible materials which can contain the neoalkyl triol triesters of this invention are bakery products, dairy products, meat products and substitutes, margarines, flavored spreads and dips, salad dressings, puddings and pie fillings, frozen desserts, candies, and the like. In these, all or a portion of the usual fat is replaced by compounds of the invention or by a mixture of Compounds of this invention.

The *tris*hydroxymethyl lower alkane esters of this invention are partially digestible, and typically provide 0.5 to 8.5 kcal/gram, more narrowly 1 to 5, and even more narrowly 1 to 3 kcal/gram upon being metabolized.

This invention comprises neoalkyl triol triester compounds of the formula:

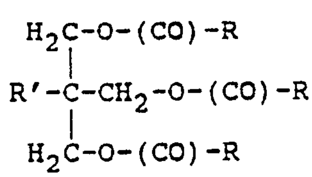

where

R′ is a lower alkyl group having 1 to 5 carbons, and each R is, independently, an aliphatic group having 1 to 29 carbons.

By "lower alkyl group" (R′) is meant an aliphatic group having 1 to 5 carbons and includes methyl ($CH_3$-), ethyl ($CH_3CH_2$-), propyl (normal, $CH_3CH_2CH_2$-, or iso, $CH_3$-$CH(CH_3)$-), butyl ($CH_3(CH_2)_3$-, or other isomer), or pentyl ($CH_3(CH_2)_4$-, or other isomer). Preferred compounds have a methyl or ethyl group as R′.

By "aliphatic group having 1 to 29 carbons" (R) is meant an aliphatic group that is saturated or unsaturated, with straight or branched chains. Typical aliphatic groups are derived from fatty acids having 2 to 30 carbons.

By the term "fatty acids" is meant synthetic or natural organic acids of the formula RCOOH. Examples of fatty acids are acetic, propionic, butyric, caproic, caprylic, pelargonic, capric, undecanoic, lauric, myristic, palmitic, stearic, arachidic, behenic, lignoceric, cerotic, montanic, melissic, palmitoleic, oleic, vaccenic, linoleic, linolenic, eleostearic, arachidonic, nervonic, eicosapentaenoic, docosatetraenoic, docosapentaenoic, and docosahexaenoic acids. Mixtures of fatty acids may also be used, for example, those derived from non-hydrogenated, partially hydrogenated or hydrogenated oils such as soybean, safflower, sunflower, sesame, peanut, corn, olive, rice bran, canola, babassu nut, coconut, palm, palm kernel, lupin, nasturtium seed, mustard seed, cottonseed, low erucic rapeseed, butter or marine oils. Fatty acids derived from plant waxes such as jojoba may also be used. Specific fractions of natural or processed oils or waxes may also be used.

The R groups are selected to provide a discernible fatty character in the compounds. Typically, most of the R groups have 3 or more carbon atoms, with a percentage containing 3 to 23 (derived from acids having 4 to 24 carbons), more narrowly 9 to 19, and even more narrowly, 13 to 17 carbons (derived from acids having 16 to 18 carbons). Preferred *tris*hydroxymethyl esters can have an array of R groups selected to include 95% having 13 to 17 carbon atoms. In one embodiment, the R groups should predominantly be in the range of 13 to 17 carbon atoms and be saturated. In another embodiment, the R groups should be predominantly in the range of 15 to 17 carbon atoms and be unsaturated (with a preponderance of monounsaturated groups).

The trizhydroxymethyl lower alkane esters of this invention are derived from neoalkyl triols of the formula:

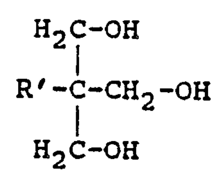

where
R′ is a lower alkyl group having 1 to 5 carbons.

Nonlimiting example neoalkyl triols are *tris*hydroxymethyl ethane (2-hydroxymethyl-2-methyl-1,3-propanediol, also called trimethylol ethane), *tris*hydroxymethyl propane (2-ethyl-2-hydroxymethyl-1,3-propanediol, also called trimethylolpropane), and *tris*hydroxymethyl butane (2-hydroxymethyl-2-propyl-2,3-propanediol, also called trimethylolbutane). Mixtures of triols may also be used.

In the practice of this invention, neoalkyl triols are condensed with fatty acids. The total amount of fatty acid present in the reaction mixture can be a maximum that theoretically will react to completely esterify all three free hydroxyls of the *tris*hydroxymethyl alkane. Thus, the total amount of fatty acid or fatty acid mixture present will be three moles for each mole of neoalkyl triol. However, best results are achieved by using an excess of fatty acid or fatty acid mixture. Typically, a three to ten per cent excess is used, preferably five per cent.

The condensation, conducted in a substantially molten state without solvents, may be described by the reaction:

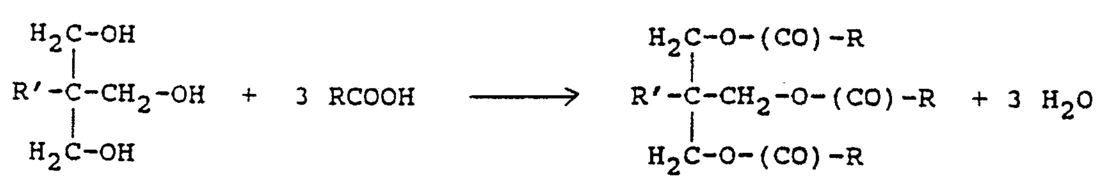

where R and R′ are as defined above.

The reactants are placed together, and heated until the reaction is substantially complete. Agitation is optional, although generally desirable. Although the reaction takes place in the absence of solvents, fatty acids from natural or processed oils may contain small amounts of residual solvent which can be removed when the products of the reaction are purified (as described below).

Application of heat simultaneously removes the water eliminated. Reaction temperatures can lie in the range of 75 to 260 ° C. Application of a vacuum or an inert gas sparge may be used to drive the reaction. Excessive temperatures are not always advantageous, and may result in codistillation of individual reactants from the mixture. It may be advantageous to begin the reaction at a lower temperture and subsequently complete the reaction at a higher temperature. The length of reaction time varies with the reaction conditions and particular reactants and may require several hours. In a temperature range of 160 to 220 ° C, for example, the esterification is substantially complete in 2 to 20 hours. The reaction time may be shortened by applying a vacuum sufficient to promote removal of water (e.g., 0.1 to 0.8 atmospheres) or sparging with an inert gas (e.g., nitrogen or carbon dioxide). Hydratable silica/alumina framework solids can be used to complete the reaction by removing water (and also serve as catalyst).

A suitable catalyst may optionally be employed, but the reaction can proceed without it. Such catalyst may either be soluble (homogeneous) or substantially insoluble (heterogeneous). Nonlimiting examples of catalysts include hydrochloric acid, phosphoric acid, polyphosphoric acid, trifluoromethanesulfonic acid, sodium sulfate, sodium bisulfite, potassium bisulfite, and zinc oxide. Additional catalysts include, but are not limited to, sulfonated and polysulfonated starch, cellulose, oligosaccharides, proteins, macroretricular solids, organic polymers (including polystyrene), and membrane films; phosphorylated and polyphosphorylated starch, cellulose, oligosaccharides, proteins, polyhydroxy-containing organic polymers and co-polymers; and carboxylated and polycarboxylated organic compounds, organic polymers, co-polymers and membrane films. Ideally, a catalyst for food use will impart no toxic properties.

After the reaction is substantially complete, the resulting *tris*hydroxymethyl alkane ester may be purified using standard edible oil refining technology. This includes conventional distillation, filtration, physical refining, alkali refining, bleaching, deodorization, and the like.

## EXAMPLES

The following examples are presented to further illustrate and explain the present invention and should not be taken as limiting in any regard. Unless otherwise indicated, all parts and percentages are by weight, and are based on the weight at the particular stage of the processing being described.

### Example 1

In this example, *tris*hydroxymethyl ethane trimyristate is synthesized in a solvent-free, catalyst-free media.

An open beaker is charged with 36 g myristic acid and 6 g *tris*hydroxymethyl ethane. The beaker is heated to 200 ° to 220 ° C (by placing in an oil bath maintained at that temperature range). The reaction mixture is sampled at time intervals to ascertain the extent of completion of the reaction as it proceeds from esterification of the triol to form, successively, monoester, diester, and, finally, triester. The results over 135 minutes are summarized in the Figure. At this point, the reaction mixture is 25 mole percent diester and 75 mole percent triester and appears to be approaching an apparent equilibrium

### Example 2

In this example, *tris*hydroxymethyl ethane trimyristate is synthesized in the presence of Amberlyst, a macroreticular, high surface area, acidic anion exchange resin, which can be regenerated after use by washing with aqueous hydrochloric acid.

A three-neck flask equipped with thermometer adapter and thermometer, manometer, and condenser tube connected to a bent vacuum adapter and receiving flask is charged with 145 g myristic acid, 24 g *tris*hydroxymethyl ethane and 13 g Amberlyst X1010. The reaction mixture is heated at 134 ° C and

vigorously stirred under vacuum (70 to 100 torr) for 20 hours. Some myristic acid codistills with the water of reaction. The reaction mixture is cooled to 100 °C and suction filtered through #4 Whatman (fast) paper to yield the triester product (uncontaminated by the diester or monoester) which can be further purified by molecular distillation and then deodorization.

## Example 3

In this example, the synthesis of *tris*hydroxymethyl ethane trimyristate in the presence of Amberlyst (described in Example 2) is further enhanced by the addition of molecular sieves after the initiation of the reaction.

A reaction flask set up as described in Example 2 is charged with 92 g myristic acid, 13 g *tris*hydroxymethylethane, and 13 g Amberlyst-X1010. The reactants are mixed and heated to 130-140 °C under vacuum (100 torr) for one and a quarter hours. At this time, 50 g activated (by heating at 200 °C overnight) 3A molecular sieves, a zeolite having an open-network structure used for selective occlusion of water, are introduced into the reaction vessel. Heating and vacuum are continued for another one and a quarter hours. Suction filtration through fast filter paper yields a slightly cloudy product which is refiltered through silica gel for further clarification before purification by the usual techniques.

## Example 4

In this example, a mixed triester of *tris*hydroxymethyl ethane is prepared.

Fatty acids derived from high oleic sunflower oil (1000 g) and 100 g (ca.0.83 mole) *tris*-hydroxymethylethane dissolved in a minimum amount of water are combined with stirring. Application of vacuum and gentle warming result in the removal of most of the water and gives a well dispersed slurry. Amberlyst XN-1010 (described in Example 2), 50 g, is added and the reaction mixture is heated between 130-135 °C for 20 hours. The mixture is cooled and steam deodorized to remove excess fatty acid. The final product is a dark red oil (*ca.* 93% triester and *ca.* 7% diester).

The above description is for the purpose of teaching the person of ordinary skill in the art how to practice the present invention, and it is not intended to detail all those obvious modifications and variations of it which will become apparent to the skilled worker upon reading the description. It is intended, however, that all such obvious modifications and variations be included within the scope of the present invention.

## Claims

1. A process for synthesizing edible *tris*hydroxymethyl lower alkane esters of the formula:

$$\begin{array}{l} \quad H_2C\text{-}O\text{-}(CO)\text{-}R \\ \qquad | \\ R'\text{-}C\text{-}CH_2\text{-}O\text{-}(CO)\text{-}R \\ \qquad | \\ \quad H_2C\text{-}O\text{-}(CO)\text{-}R \end{array}$$

where

R' is a lower alkyl group having 1 to 5 carbons, and each R is, independently, an aliphatic group having 1 to 29 carbons,

comprising:

reacting a *tris*hydroxymethyl triol of the formula

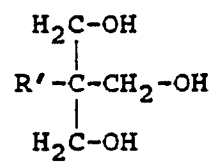

with fatty acid(s) of the formula RCOOH, where R and R′ are as defined above , the reaction mixture being heated until the reaction is substantially complete.

2. The process according to claim 1 wherein the *tris*hydroxymethyl triol is selected from *tris*hydroxymethyl ethane, trishydroxymethyl propane, *tris*hydroxymethyl butane, and mixtures thereof.

3. The process according to claim 1 wherein R′ is methyl ($CH_3$-) or ethyl ($CH_3$-$CH_2$-).

4. The process according to any preceding claim wherein R contains 3 to 23 carbon atoms.

5. The process according to claim 4 wherein R contains 13 to 17 carbon atoms.

6. The process according to any one of claims 1-3 wherein the fatty acid(s) are selected from acetic, propionic, butyric, caproic, caprylic, pelargonic, capric, undecanoic, lauric, myristic, palmitic, stearic, arachidic, behenic, lignoceric, cerotic, linolenic, elestearic, arachidonic, nervocenic, linoleic, linolenic, eleostearic, arachidonic, nervonic, eicosapentaenoic, docosatetraenoic, docosapentaenoic, and docosahexaenoic acids, and mixtures thereof.

7. The process according to any one of claims 1-3 wherein the fatty acid(s) are derived from non-hydrogenated, partially hydrogenated and fully hydrogenated oils selected from soybean, safflower, sunflower, sesame, peanut, corn, olive, rice bran, canola, babassu nut, coconut, palm, palm kernel, lupin, nasturtium seed, mustard seed, cottonseed, low erucic rapeseed, butter, and marine oils, and fractions thereof.

8. The process according to any preceding claim wherein the reaction is conducted in the presence of an acid catalyst.

9 The process according to claim 8 wherein the acid catalyst is selected from hydrochloric acid,
phosphoric acid, triflurormethane sulfonic acid, potassium bisulfite, sodium bisulfite, zinc oxide, and sulfonated polystyrene.

10. The process according to claim 8 wherein the acid catalyst is an anion exchange resin.

11. The process according to any preceding claim wherein an hydratable silica/alumina framework solid is used to complete the reaction.

12. The process according to claim 11 wherein the framework solid is a zeolite that selectively occludes water.

13. A process for preparing a partially digestible edible fat mimetic product comprising:
reacting *tris*hydroxymethyl ethane or *tris*hydroxymethyl propane with fatty acid(s), the reaction mixture being warmed until the reaction is substantially complete.

14. The process according to claim 13 wherein the reaction is conducted in the presence of an acid catalyst selected from hydrochloric acid, phosphoric acid, trifluoromethane sulfonic acid, potassium bisulfite, sodium bisulfite, sodium sulfate, zinc oxide, and sulfonated polystyrene.

15. The process according to claim 13 or 14 wherein the fatty acid(s) comprise $C_2$ to $C_{30}$ fatty acid(s).

16. The process according to claim 15 wherein the fatty acid(s) comprise $C_3$ to $C_{23}$ fatty acid(s).

17. A substantially solvent-free, catalyst-free process for synthesizing edible fatty acid triesters of *tris*hydroxy methyl lower alkane esters of the formula:

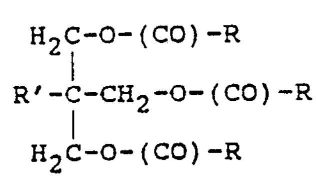

where
R′ is a lower alkyl group having 1 to 5 carbons, and each R is, independently, an aliphatic group having 1 to 29 carbons,
comprising:
reacting a trizhydroxymethyl triol of the formula

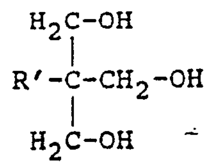

with fatty acid(s) of the formula RCOOH, where R and R′ are as defined above, the reaction mixture being heated until the reaction is substantially complete.

18. The process according to claim 17 wherein the *tris*hydroxymethyl lower alkane is selected from *tris*hydroxymethyl ethane, trishydroxymethyl propane, and *tris*hydroxymethyl butane, and mixtures thereof.

19. The process according to claim 17 or 18 wherein the fatty acid(s) are selected to produce a discernible fatty character in the compounds.

20. The process according to claim 19 wherein the fatty acid(s) comprise $C_3$ to $C_{23}$ fatty acid(s).

21. The process according to claim 20 wherein the fatty acid(s) comprise $C_{13}$ to $C_{17}$ fatty acid(s).

22. The process according to claim 21 wherein the fatty acid(s) are saturated.

23. The process according to claim 20 wherein the fatty acid(s) comprise $C_{15}$ to $C_{17}$ fatty acid(s).

24. The process according to any one of claims 17-23 which further comprises driving the reaction by removing water with an hydratable silica/alumina framework solid.

25. The process according to claim 24 wherein the solid is a zeolite that occludes water.

26. A partially digestible edible fat mimetic product prepared by the process of

reacting $C_2$ to $C_{30}$ fatty acid(s) with neoalkyl triol(s) selected from trishydroxymethyl ethane, *tris*hydroxymethyl propane, *tris*hydroxymethyl butane, and *tris*hydroxymethyl pentane, and mixtures thereof, the reaction mixture being heated until the reaction is substantially complete.

27. The product according to claim 26 wherein the fatty acid(s) are selected from acetic, propionic, butyric, caproic, caprylic, pelargonic, capric, undecanoic, lauric, myristic, palmitic, stearic, arachidic, behenic, lignoceric, cerotic, montanic, melissic, palmitoleic, oleic, vaccenic, linoleic, linolenic, eleostearic, arachidonic, nervonic, eicosapentaenoic, docosatetraenoic, docosapentaenoic, and docosahexaenoic acids, and mixtures thereof.

28. The product according to claim 26 wherein the fatty acid(s) are derived from non-hydrogenated, partially hydrogenated and hydrogenated oils selected from soybean, safflower, sunflower, sesame, peanut, corn, olive, rice bran, canola, babassu nut, coconut, palm, palm kernal, lupin, nasturtium seed, mustard seed, cottonseed, low erucic rapeseed, butter, and marine oils, and fractions thereof.

29. The product according to claim 26 which delivers 0.5 to 8.5 kcal/gram upon being metabolized.

30. The product according to to any one of claims 26-29 wherein the process further comprises using an acid catalyst.

31. A food composition comprising a partially digestible edible fat mimetic compound prepared by the process of

reacting $C_2$ to $C_{30}$ fatty acid(s) with neoalkyl triol(s) selected from trishydroxymethyl ethane, *tris*hydroxymethyl propane, *tris*hydroxymethyl butane, and *tris*hydroxymethyl pentane, and mixtures thereof, the reaction mixture being heated until the reaction is substantially complete.

32. The composition of claim 31 wherein the fatty acid(s) comprise $C_4$ to $C_{24}$ acids and the neoalkyl triols are selected from trishydroxymethyl ethane and *tris*hydroxymethylpropane, and mixtures thereof.

33. The composition of claim 31 or 32 wherein said food composition is a bakery product.

34. The composition of claim 31 or 32 wherein said food composition is a dairy product.

35. The composition of claim 31 or 32 wherein said food composition is a salad dressing.

36. A method for preparing edible *tris*hydroxymethyl ethane fatty acid ester(s)which comprises heating *tris*hydroxymethyl ethane with fatty acid(s) until esterification is substantially complete.

37. A method for preparing edible *tris*hydroxymethyl propane fatty acid esters which comprises heating *tris*hydroxymethyl propane with fatty acid(s) until the esterification is substantially complete.

38. An improvement in the synthesis of edible *tris*hydroxy methyl ethane and *tris*hydroxymethyl propane fatty acid esters which comprises esterifying *tris*hydroxymethylethane and *tris*hydroxymethyl propane with fatty acid(s) in the absence of solvents and catalysts.

39. A solvent-free, catalyst-free process for synthesizing edible *tris*hydroxymethyl lower alkane esters comprising heating one molar equivalent of *tris*hydroxymethyl lower alkane selected from trishydroxy methyl ethane and *tris*hydroxymethyl propane with a 5% excess over three molar equivalents of fatty acid to a temperature of 100 to 260 °C until the reaction is substantially complete.

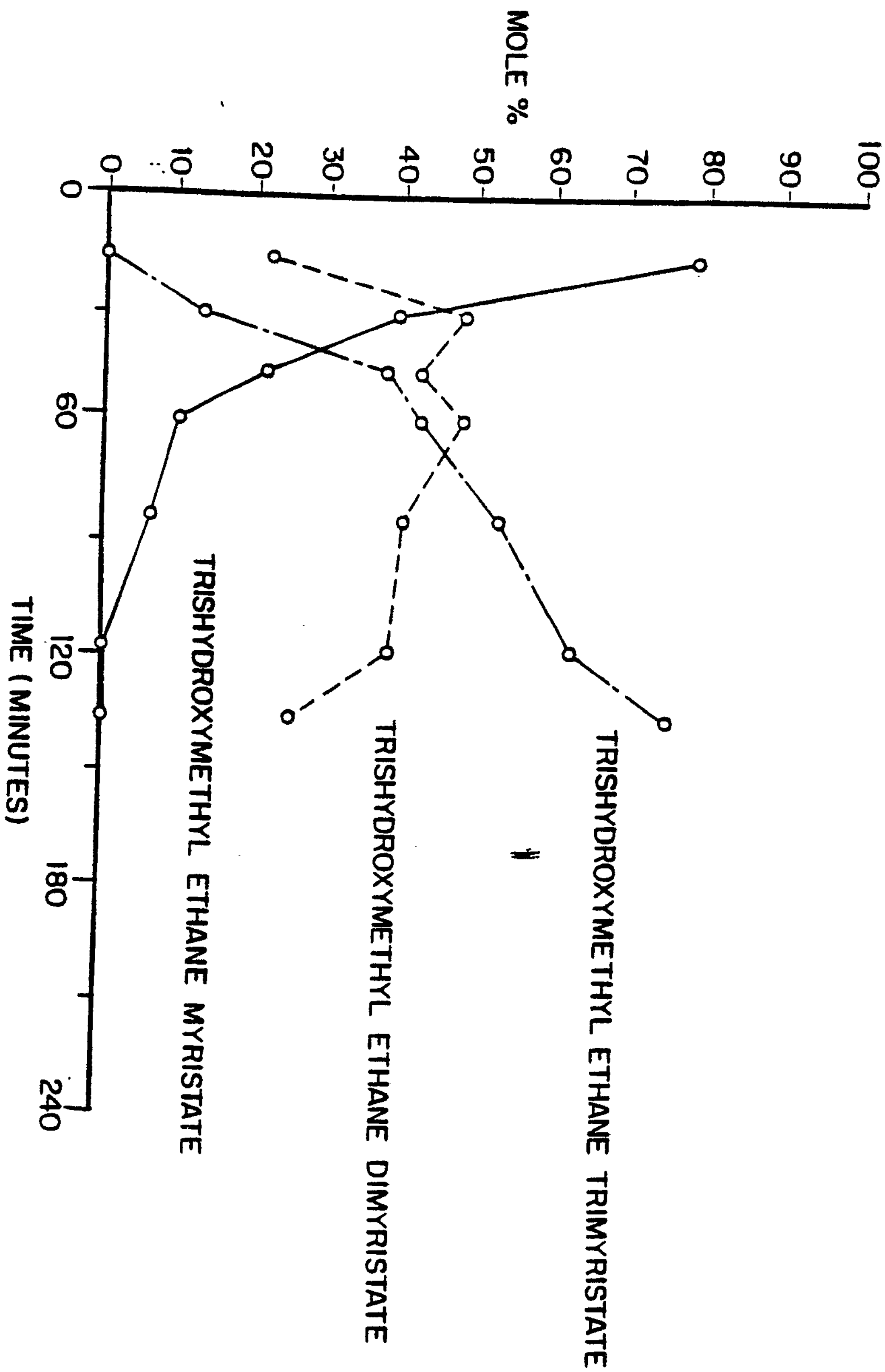
MOLE %
0
10
20
30
40
50
60
70
80
90
100
TIME (MINUTES)
0
60
120
180
240
TRISHYDROXYMETHYL ETHANE TRIMYRISTATE
TRISHYDROXYMETHYL ETHANE DIMYRISTATE
TRISHYDROXYMETHYL ETHANE MYRISTATE